(19) Europäisches Patentamt
European Patent Office
Office européen des brevets

(11) EP 4 714 940 A1

(12) EUROPEAN PATENT APPLICATION

(43) Date of publication:
**25.03.2026 Bulletin 2026/13**

(21) Application number: **24383019.7**

(22) Date of filing: **23.09.2024**

(51) International Patent Classification (IPC):
***C07D 207/16*** *(2006.01)* ***A61K 31/401*** *(2006.01)*
***A61P 9/10*** *(2006.01)* ***A61P 11/00*** *(2006.01)*
***A61P 11/06*** *(2006.01)* ***A61P 19/02*** *(2006.01)*
***A61P 21/00*** *(2006.01)* ***A61P 25/04*** *(2006.01)*
***A61P 25/08*** *(2006.01)* ***A61P 25/18*** *(2006.01)*
***A61P 25/24*** *(2006.01)* ***A61P 25/28*** *(2006.01)*
***A61P 25/30*** *(2006.01)* ***A61P 31/00*** *(2006.01)*
***A61P 35/00*** *(2006.01)*

(52) Cooperative Patent Classification (CPC):
**C07D 207/16; A61K 31/401; A61P 9/10; A61P 11/00; A61P 11/06; A61P 19/02; A61P 21/00; A61P 25/04; A61P 25/08; A61P 25/18; A61P 25/24; A61P 25/28; A61P 25/30; A61P 31/00; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Accure Therapeutics, S.L.**
**08028 Barcelona (ES)**

(72) Inventor: **PRADES COSANO, Roger**
**E-08028 Barcelona (ES)**

(74) Representative: **ABG Intellectual Property Law, S.L.**
**Avenida de Burgos, 16D**
**Edificio Euromor**
**28036 Madrid (ES)**

# (54) GELATINASE INHIBITORS AND USE THEREOF

(57) New gelatinase inhibitors, processes for their preparation, pharmaceutical compositions comprising them, and their use in therapy and/or prophylaxis of conditions wherein inhibition of gelatinases is useful such as epilepsy, schizophrenia, Alzheimer disease, autism (in particular associated to fragile X syndrome), mental retardation, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

EP 4 714 940 A1

**Description**

## FIELD OF THE INVENTION

The present invention relates to gelatinase inhibitors, to processes for their preparation, to pharmaceutical compositions comprising them, and to their use in therapy and/or prophylaxis of conditions wherein inhibition of gelatinases is useful such as epilepsy, schizophrenia, Alzheimer disease, autism (in particular associated to fragile X syndrome), mental retardation, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

## BACKGROUND

There are accumulated evidences that the extracellular matrix (ECM) not only acts as a support structure but also has considerable effects on neuronal development and regeneration, synaptic plasticity, neuronal excitability, and homeostatic regulations of network activity. Indeed, the ECM has a profound impact on network behavior, hence on physiological processes such as cognition. Matrix metalloproteinases (MMPs) are a family of zinc dependent endopeptidases capable to degrade ECM proteins as well as a large number of non-ECM proteins, such as growth factors, cytokines, chemokines, cell surface receptors, serine proteinase inhibitors and other MMPs. MMPs are either secreted or membrane bound proteases and play major physiological roles in reproduction, growth, development, angiogenesis, immune response, wound healing and brain physiology. There is enhanced expression of MMPs, in particular gelatinases (MMP-2 or gelatinase A and MMP-9 or gelatinase B), during numerous pathological conditions, including tumor progression, neurodegeneration, stroke, inflammation and viral infections.

Initial studies on MMP-9 in the brain originally were focused on its possible role in a variety of pathological conditions with a degenerative component. More recently, MMP-9 has emerged as an important player in the brain physiology, especially as being a key molecule in the synaptic plasticity. These findings led to studies demonstrating a possible involvement of MMP-9 in neuropsychiatric conditions such as epilepsy, schizophrenia, Alzheimer disease, autism (in particular associated to fragile X syndrome), mental retardation, bipolar disorders, mood disorders such as bipolar disorders, depression and drug addiction.

Alterations in glutamate signaling, leading to aberrant synaptic plasticity, in schizophrenia have been described. MMP-9 has been shown to regulate glutamate receptors, be regulated by glutamate at excitatory synapses, and modulating physiological and morphological synaptic plasticity. By means of functional gene polymorphism, gene responsiveness to antipsychotics and blood plasma levels MMP-9 has recently been implicated in schizophrenia. This disease involves impairments in perception and cognition, culminating in a triad of positive, negative, and cognitive symptoms that are believed to reflect modifications in neuronal circuitry, the perturbation of synaptic connectivity, and alterations in dendritic spines. Notably, chromosome region 20q11-13, where the MMP-9 gene is located, has been extensively studied in terms of psychiatric disorders and linked to schizophrenia. MMP-9 influences hippocampal and prefrontal cortical function and is an interesting candidate molecule that is potentially involved in schizophrenia, a condition in which prefrontal cortex impairment is one of the most common pathological findings. Furthermore, some of the candidate proteins that are implicated in schizophrenia have functional connections with either MMP-9 or MMP-9-interacting proteins, such as brain derived neurotrophic factor (BDNF) and N-methyl-D-aspartate (NMDA) receptors, among others. Abnormal elevated MMP-9 levels have been found in plasma of schizophrenic patients as reported by several authors (Yamamori H et al. Neurosci Lett., 2013;556:37-41).

There are accumulating evidences of the involvement of MMPs in the pathogenesis of epilepsy. This disease is a brain disorder characterized by an enduring predisposition to generate epileptic seizures and by the neurobiological, cognitive, psychological, and social consequences of this condition. It has been demonstrated that prolonged seizures are related to high-serum MMP-9 levels. More importantly, recent studies in brain tissue obtained during epilepsy surgery reported increased MMP-9 immunoreactivity in epileptogenic lesions of focal cortical dysplasia (Konopka A et al., Epilepsy Res.,2013, (1-2:45-58) and tuberous sclerosis (Li S et al., Brain Res, 2012, 1453:46-55; Broekaart DW et al., Neuropathol Appl Neurobiol., 2020, 142-159) as well as in the epileptogenic cortical or hippocampal lesions of patients with temporal lobe epilepsy without underlying cytoarchitectonic abnormalities. Using an unbiased approach of antibody microarrays it has been found an elevated expression of MMP-1, -2, -3, -8, - 10 and -13, in addition to MMP-9, in the tissue from patients with focal cortical dysplasia (Konopka A et al., Epilepsy Res., 2013, 1-2:45-58). However, the expression of these proteinases was not as pronounced and/or not as consistent among patients as the expression of MMP-9. Among these other MMPs, especially striking was the upregulation of MMP-2. High levels of MMP-9 are also observed after traumatic brain injury promoting the development of posttraumatic epilepsy (Pijet B et al., Mol Neurobiol., 2018, 55:9294-9306).

Autism spectrum disorders (ASDs) are identified by a cluster of symptoms in three core domains: social interaction, language, and range of interests, but in most cases their etiology is unknown. Fragile X syndrome (FXS)

is the leading genetic cause of autism since a large percentage of individuals with FXS (46%) are co-diagnosed with ASD. High plasma activity of MMP-9 has been reported in individuals with FXS (Leigh M J et al, J Dev Behav Pediatr, 2013, 34:1849-1857), whereas elevated protein amounts of MMP-9 were detected in amniotic fluid from ASD mother (Abdallah M W et al, Autism Res, 2012, 5:428-433). Therefore, a clear connection exists between high levels ofMMP-9 and ADS.

MMP-9 has been implicated also in human drug addiction, bipolar disorder and depression. One link is provided due to analyses of MMP-9 gene polymorphism at -1562 that is functional, as it results in higher or lower MMP-9 expression. It has been reported that frequently of this polymorphism differentiates between healthy subjects and patients suffering from either bipolar disorders or schizophrenia (Han H et al, Psychiatry Res, 2011, 190:163-174). This polymorphism has been linked as well to alcohol addiction (Samochowiec A et al, Brain Res, 2010, 1327:103-6). In addition to this, it has been found that MMP-9 gene polymorphism modulates prefrontal cognition in bipolar men. Increased MMP-9 levels in young patients during bipolar depression have been reported (Rybakowski J K et al, J Affect Disord, 2013, (146):286-289). High levels of MMP-9 in plasma have been detected in depression, this serum levels correlate with the severity of the depression (Yoshida T et al, PLoS One, 2012, 7:e42676).

MMP-9 is also claimed to be linked to degenerative diseases such Alzheimer's disease (AD). It has been shown that MMP participates in the formation and clearance of the Aβ peptides in AD. In fact, increased levels of MMP-9 have been observed in the brain tissue and blood of patients with AD, in particular in reactive astrocytes surrounding amyloid plaques, suggesting a local tissue response to plaque accumulation. Several studies have documented that this metalloproteinase participates in Aβ catabolism in vitro and in vivo and it is the only enzyme capable of degrading Aβ fibrils in vitro and Aβ plaques in situ. In addition, it has been reported that MMP-9 is involved in receptor-mediated sAPP-α release and exhibits a α-secretase-like activity in the brain in vivo (Fragkouli A et al, J Neurochem, 2012, 121:239-251). Although potentiating MMP activity in AD may be advantageous, contradictory results challenge this idea. One study has shown that Aβ-induced activation of MMP-2 in human cerebrovascular smooth muscle contributes to cell death, loss of vessel wall integrity, and hemorrhagic stroke in cerebral amyloid angiopathy (CAA). Moreover, the inhibition of MMPs with minocycline, simvastin, or GM6001 decreases inflammation and oxidative stress associated with CAA in AD mice. Seemingly, synthetic inhibitors of MMP-2/MMP-9 reduce Aβ-mediated neuronal death in primary cultures (Mizoguchi et al, J Pharmacol Exp, 2009, (331):14-22). In the same study, GM60001 treatment was neuroprotective upon intracerebroventricular administration of Aβ and improved cognition in mice. Moreover, MMP-9 KO mice did not undergo the memory deficits induced by Aβ injections in wild-type mice.

MMP-9 has also been identified to be involved in process and disease conditions other than those related with synaptic plasticity. These conditions include vascular, lung and inflammatory diseases, and cancer. In this regard, MMP-9, and in a lesser extend MMP-2, have been linked to vascular diseases such as ischemic stroke and atherosclerosis, neuropathic pain, inflammatory diseases such as multiple sclerosis and rheumatoid arthritis, multiple sclerosis, sepsis, cancer, lung diseases such as asthma and chronic obstructive pulmonary disease.

Ischemic stroke is a consequence of a deficit of a blood supply (resulting from a local thrombosis or arterial embolism), producing a decreased tissue oxygenation and in consequence bioenergetics disturbances that may lead to cell death of both necrotic and apoptotic character. Restoration of blood circulation after temporary hypoperfusion results in a robust inflammatory response that may exacerbate the tissue damage. The accumulation of inflammatory cells is then responsible for the high levels of reactive oxygen and nitrogen species as well as proinflammatory cytokines in the ischemic tissue.

A dramatic increase of MMP-9 at all levels of its expression and activity is a hallmark of stroke consequences. MMP-9 is involved in such post-stroke events as long-term plasticity, vascular reorganization and angiogenesis, immune response and inflammatory. Blocking the MMPs, MMP-9 in particular, including KO mice, has been demonstrated to be protective against ischemic stroke and its consequences (Chaturvedi M et al., Mol. Neurobiol., 2014, 49:563-573).

Treatment of neuropathic pain, triggered by multiple insults to the nervous system, is a clinical challenge because the underlying mechanism of neuropathic pain development remains poorly understood. However, recent studies report that early- and late- phase neuropathic pain development in rats and mice after nerve injury require different MMP. After spinal nerve injury, MMP-9 shows a rapid and transient upregulation in injured dorsal root ganglion (DRG) primary sensory neurons consistent with an early phase of neuropathic pain, whereas MMP-2 shows delayed response in DRG satellite cells and spinal astrocytes consistent with a late phase of neuropathic pain. Intrathecal administration of MMP-9 inhibitors or TIMP-1, an endogenous tissue inhibitor of MMP-9, delay the development of mechanical allodynia (central pain sensitization following painful stimulation) the first days (<10) after the injury. However, the inhibition of MMP-9 has no effect on allodynia when given 10 days after brain injury, showing the critical role of MMP-9 in the early development of neuropathic pain. Compared to MMP-9, MMP-2 upregulation after spinal nerve injury shows a delayed pattern. Intrathecal administration of TIMP-2, an endogenous inhibitor of MMP-2, or small synthetic inhibitors of MMP-2 partly attenuates allodynia on day 1 after injury but almost completely block allodynia in the following ten days. This shows the involvement of MMP-2 in the late phase of neuropathic pain. (Kawasaki Y et al, Nature Medicine, 2008, 3:331-336).

During an inflammatory response, leukocyte traffic through tissue barriers, including basement barriers membranes, is only possible if these cells are equipped with enzymes that can remodel ECM. MMP are therefore crucial effector

molecules of inflammatory cells. MMPs can act as switches or as delicate turners in acute and chronic inflammation, during autoimmune diseases, when triggered in vascular diseases and in the regenerative phase after inflammation. Thus, MMP biology is important in the definition, execution and resolution phases of acute and chronic inflammatory and ischemic processes and consequently, MMP inhibitors might interfere with these. MMP inhibitors have been tested in many animal models of acute and chronic inflammation, such endotoxin shock, multiple sclerosis and rheumatoid arthritis. Bacteraemia, septic and endotoxin shock are the most frequent causes of mortality in modern hospitals. Bacterial cell-wall constituents induce a systematic response by the activation of the toll-like receptors, leading to an excessive production of inflammatory cytokines and enzymes. Mice deficient in MMP-9 have an altered resistance bacterial induced toxicity, whereas mice deficient in protease inhibitors are more susceptible to endotoxin shock. In multiple sclerosis is a multifactorial disease that is influenced by genetic predisposition, environmental factors and immunological effector mechanisms that damage the central nervous system. MMP-9 is an immune effector molecule in multiple sclerosis (Opdenakker G et al., Lancet Nurol., 2003, 2:747-756). It functions in cell migration through connective tissues and vessel walls and damages the blood-brain barrier. It also lyses protein substrates, such as myelin proteins, cell-adhesion molecules, cytokines and chemokines that are relevant in multiple sclerosis and other neurological diseases. Evidences which supports a detrimental role of MMP-9 in inflammatory CNS damage has been obtained in animal models. Murine experimental autoimmune encephalomyelitis (EAE), a model for multiple sclerosis, both gelatinases MMP-2 and MMP-9, become upregulated during the development of the disease syndrome (Gijbels k et al., J. Neurosci. Res., 1993, 36:432-440). Young MMP-9 deficient mice have resistance to the development of EAE. Double Mmp2/Mmp9-knockout mice are completely resistant against the development of EAE. Pharmacological inhibition of MMP activity improves the course of EAE in several studies that used MMP inhibitors (Hewson A et al., Inflamm. Res., 1995, 44:345-349). The use of MMP inhibitors might be also useful in the therapy of rheumatoid arthritis. MPP-9 is involved in the degradation of collagen II during rheumatoid arthritis, leading to the exposure and release of immunodominant epitopes. In addition, MMPs are important for the migration of inflammatory leukocytes. This suggests that MMP inhibitors might be useful in the therapy of rheumatoid arthritis, a notion that has been confirmed in different animals models (Agrawal S et al., J. Exp. Med., 2006, 203:1007-1019).

Atherosclerosis and related diseases, including myocardial infarction and stroke, have often been compared with chronic inflammatory diseases. This is based on histopathological findings such as the activation of foamy macrophages, the local production of cytokines and chemokines, and the involvement of MMPs. The use of animal models with genetically altered mice (both transgenic and knockout) has strengthened the view that MMPs are key players in vascular pathologies (Janssens S et al., Cardiovsac. Res., 2006, 69:585-594 and Tayebjee M H et al., Curr. Med. Chem., 2005, 12:917-925). It has been reported that MMP-9 levels increase with the progression of idiopathic atrial fibrillation (Li M et al., J. Int. Med. Res., 2014, 1:224-230) and is associated with the development of aortic aneurysms [Newman K M et al., Arteriosclerosis and thrombosis: a journal of vascular biology, 1994, 8:1315-1320)]. Inhibition of MMP-9 suppresses the growth of aortic aneurysms (Lindeman J H et al., Circulation, 2009, 119:2209-2216). Sudden death after myocardial infarction can occur by cardiac rupture, a process in which MMPs are involved. In mouse studies, the critical role of gelatinases in balance with endogenous tissue inhibitors of metalloproteases (TIMPs) is demonstrated. Myocardial infarction could be reversed by treating mice with an oral inhibitor of MMP-2 (Matsumura S et al., J. Clin. Invest., 2005, 115:559-609).

Mortality in cancer is primarily because of failure to prevent metastasis. Emerging evidences has emphasized the role of MMPs in early aspects of cancer dissemination (Kessenbrock K et al., Cell, 2010, 141:52-67). Enzymes that degrade the ECM have long been viewed as essential for tumor progression. Tumor cells are envisioned to produce enzymes that destroy the matrix barriers surrounding the tumor, permitting invasion into surrounding connective tissues, entry and exit from blood vessels, and metastasis to distant organs. MMPs have the capacity to degrade all structural components of ECM. Moreover, MMPs are up-regulated in virtually all human and animal tumors as well as in most tumor cell lines (Coussens L M et al., Science, 2002, 295:2387-2392). MMP-9 is linked to cancer invasion. Elevated levels of MMP-9 in tissue and blood are observed in cancer patients, thus making MMP-9 attractive targets for cancer therapy, since the ability of MMP-9 to degrade collagen and laminin correlates with its ability to regulate cell migration, increase angiogenesis and tumor growth (Bjorklund M et al., Biochim Biophys Acta, 2005, 1755:37-69).

In pathological lung conditions, MMPs and their physiological inhibitors (TIMPs) and abnormally over expressed and produced in the respiratory tract by a panel of different structural cells. Alternations in these biological activities have several dramatic effects in wound healing and cell trafficking. Deregulation of various MMPs by stimulated structural or inflammatory cells is thought to take part to pathophysiology of numerous lung diseases including asthma, chronic obstructive pulmonary disease (COPD), lung fibrosis and lung cancer (Demedts I K et al, Curr Opin Pharmacol, 2005, 5(3):257-63). The inflammation process is characterized by the extracellular matrix remodeling and collagen deposition that in turns request increased levels of MMP-9 (Kelly EA, et al, Am. J. Respir. Crit. Care Med., 162 (2000), pp. 1157-1161). Selective inhibition of MMP-9 is thought to promote a therapeutical benefit in these associated chronic inflammatory lung diseases as it has been proven recently in the treatment of COPD (Xie SS., et al, J Int Med Res. 2014 Dec;42(6):1272-84).

Patent document WO2017085034A1 discloses a family of MMP-2 and MMP-9 inhibitors.

## BRIEF DESCRIPTION OF THE INVENTION

The inventors have now successfully found a new family of MMP-2 and/or MMP-9 inhibitors having increased stability and safety profile when compared to prior art compounds disclosed in WO2017085034A1, as shown in the examples. Thus, the compounds of the present invention are ideal candidates for use in the therapy of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

Therefore, the first aspect of the invention relates to a compound of formula (I):

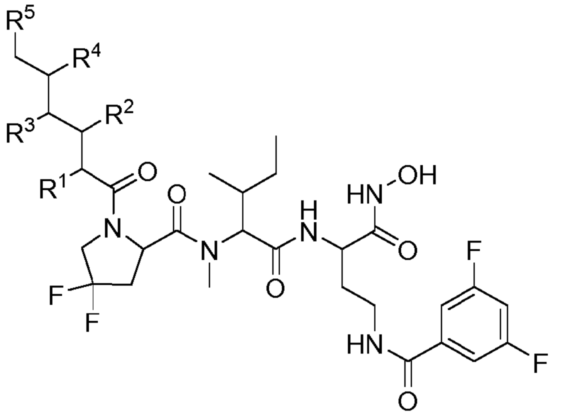

(I)

wherein

each one of $R^1$, $R^2$, $R^3$, $R^4$ and $R^5$ is independently selected from a hydrogen atom and a hydroxyl group, with the proviso that at least one of $R^1$, $R^2$, $R^3$, $R^4$ and $R^5$ is a hydroxyl group, or a pharmaceutically acceptable salt thereof or stereoisomer thereof.

A second aspect of this invention refers to pharmaceutical compositions comprising a compound of formula (I) as defined in the first aspect and a pharmaceutically acceptable carrier, adjuvant or vehicle.

A third aspect of this invention refers to a compound of formula (I) as defined in the first aspect, or a pharmaceutical composition as defined in the second aspect, for use as a medicament, particularly for the prevention and/or treatment of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

Another aspect of this invention refers to a method for the treatment or prophylaxis of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis in a mammal wherein a therapeutic amount of a compound of formula (I) as defined in the first aspect, or a pharmaceutical composition as defined in the second aspect, is administered to a patient in need of said treatment.

Another aspect of this invention refers to the use of a compound of formula (I) as defined in the first aspect, or a pharmaceutical composition as defined in the second aspect, for the preparation of a medicament, particularly for the prevention and/or treatment of a disease selected from the group consisting of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis. These aspects and preferred embodiments thereof are additionally also defined in the claims.

## DETAILED DESCRIPTION OF THE INVENTION

In the context of the present invention some abbreviations and acronyms have been use and their meanings are provided below:

ACN: Acetonitrile
Alloc: N-Allyloxycarbonyl
Clint: Intrinsic clearance
Dab: Diaminobutyric
DAD: Diode Array Detector
DBU: 1,8-Diazabicyclo[5.4.0]undec-7-ene
DCM: Dichloromethane
DDI: Drug-Drug Interaction
DIC: *N,N*-diisopropylcarbodiimide
DIEA: *N,N*-Diisopropylethylamine
DIPCDI: *N,N'*-Diisopropylcarbodiimide
DMAP: 4-(Dimethylamino)pyridine
DMF: Dimethylformamide
DMSO: Dimethyl sulfoxide
Fmoc: Fluorenylmethyloxycarbonyl
HFIP: Hexafluoro-2-propanol
HEP: Hepatocyte
HLM: Human Liver Microsomes
HOAt: 1-Hydroxy-7-azabenzotriazole
HPLC: High Performance Liquid Chromatography
LC: Liquid Chromatography
MeOH: Methanol
NADPH: Nicotinamide Adenine Dinucleotide Phosphate
Oxyma pure: Ethyl cyano(hydroxyimino)acetate
PyBOP: Benzotriazol-1-yloxytripyrrolidinophosphonium hexafluorophosphate rpm: Revolutions per minute
TFA: Trifluoroacetic acid
TOF: Time of Flight
UPLC-MS: Ultra Performance Liquid Chromatography - Mass Spectrometry UV: Ultraviolet

The term "salt" must be understood as any form of an active compound used in accordance with this invention in which said compound is in ionic form or is charged and coupled (associated) to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

The term "pharmaceutically acceptable salts" in the context of this invention means any salt that is tolerated physiologically (normally meaning that it is not toxic, particularly, as a result of the counter-ion) when used in an appropriate manner for the treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion, particularly when used on humans and/or mammals. These physiologically acceptable salts may also be formed with anions or acids and, in the context of this invention, are understood as being salts formed by at least one compound used in accordance with the invention -normally protonated, for example a protonated nitrogen- such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids- particularly when used on humans and/or mammals.

Pharmaceutically acceptable acids include inorganic acids, such as hydrochloric, sulphuric, phosphoric, diphosphoric, hydrobromic, hydroiodic and nitrate acids, and organic acids, such as citric, maleic, malic, mandelic, ascorbic, oxalic, succinic, tartaric, acetic, methanesulfonic, ethanesulfonic, benzenesulfonic and p-toluenosulfonic acids. Pharmaceutically acceptable bases include hydroxides of alkali metals (e.g. sodium or potassium), alkaline-earth metals (for example, calcium or magnesium) and organic bases (for example, alkylamines, arylalkyilamines and heterocyclic amines).

Other preferred salts according to the invention are quaternary ammonium compounds in which an equivalent of

an anion (X-) is associated with the positive charge of the N atom. X- may be an anion of diverse mineral acids such as for example, chloride, bromide, iodide, sulfate, nitrate, phosphate, or an anion of an organic acid, such as acetate, maleate, fumarate, citrate, oxalate, succinate, tartrate, malate, mandelate, trifluoracetate, methanesulfonate and *p*-toluenesulfonate. X- is preferably an anion selected from chloride, bromide, iodide, sulfate, nitrate, acetate, maleate, oxalate, succinate and trifluoracetate. More preferably X- is chloride, bromide, trifluoracetate or methanesulfonate.

The compounds of the present invention represented by the above described formula (I) may include enantiomers and/or diastereoisomers depending on the presence of chiral centres. The single isomers, enantiomers or diastereoisomers and mixtures thereof fall within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by $^{13}C$- or $^{14}C$-enriched carbon, or the replacement of at least one nitrogen by $^{15}N$-enriched nitrogen are within the scope of this invention.

The compounds of formula (I), or their salts are preferably in pharmaceutically acceptable or substantially pure form. By pharmaceutically acceptable form is meant, *inter alia,* having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

As noted previously, the term "pharmaceutically acceptable salts" refers to any salt which, upon administration to the recipient, is capable of providing (directly or indirectly) a compound as described herein. However, it will be appreciated that non-pharmaceutically acceptable salts of the compounds of formula (I) also fall within the scope of the invention since those may be useful in the preparation of pharmaceutically acceptable salts of said compounds. The preparation of salts can be carried out by methods known in the art.

In one embodiment of the present invention the group $R^1$ in the compounds of formula (I) is a hydroxyl group and $R^2$, $R^3$, $R^4$ and $R^5$ are selected from the group consisting of hydrogen atoms and hydroxyl groups.

In one embodiment of the present invention the group $R^2$ in the compounds of formula (I) is a hydroxyl group and $R^1$, $R^3$, $R^4$ and $R^5$ are selected from the group consisting of hydrogen atoms and hydroxyl groups.

In one embodiment of the present invention the group $R^5$ in the compounds of formula (I) is a hydroxyl group and $R^1$, $R^2$, $R^3$ and $R^4$ are selected from the group consisting of hydrogen atoms and hydroxyl groups.

In one embodiment of the present invention the group $R^3$ in the compounds of formula (I) is a hydroxyl group and $R^1$, $R^2$, $R^4$ and $R^5$ are selected from the group consisting of hydrogen atoms and hydroxyl groups.

In one embodiment of the present invention the group $R^4$ in the compounds of formula (I) is a hydroxyl group and $R^1$, $R^2$, $R^3$ and $R^5$ are selected from the group consisting of hydrogen atoms and hydroxyl groups.

In one embodiment of the present invention in the compounds of formula (I) only one of $R^1$, $R^2$, $R^3$, $R^4$ and $R^5$ is a hydroxyl group and the rest are hydrogen atoms.

In one embodiment of the present invention the group $R^1$ in the compounds of formula (I) is a hydroxyl group and $R^2$, $R^3$, $R^4$ and $R^5$ are hydrogen atoms.

In one embodiment of the present invention the group $R^2$ in the compounds of formula (I) is a hydroxyl group and $R^1$, $R^3$, $R^4$ and $R^5$ are hydrogen atoms.

In one embodiment of the present invention the group $R^5$ in the compounds of formula (I) is a hydroxyl group and $R^1$, $R^2$, $R^3$ and $R^4$ are hydrogen atoms.

In one embodiment of the present invention the group $R^3$ in the compounds of formula (I) is a hydroxyl group and $R^1$, $R^2$, $R^4$ and $R^5$ are hydrogen atoms.

In one embodiment of the present invention the group $R^4$ in the compounds of formula (I) is a hydroxyl group and $R^1$, $R^2$, $R^3$ and $R^5$ are hydrogen atoms.

In one embodiment of the present invention the groups $R^1$, $R^2$ and $R^5$ are selected from hydroxyl group and hydrogen atom, with the proviso that at least one, and preferably only one, of the groups $R^1$, $R^2$ and $R^5$ in the compounds of formula (I) is a hydroxyl group, and the groups $R^3$ and $R^4$ are hydrogen atoms.

In one embodiment, the compound of formula (I) is selected from the group consisting of:

- N-(1-((4-(3,5-difluorobenzamido)-1-(hydroxyamino)-1-oxobutan-2-yl)amino)-3-methyl-1-oxopentan-2-yl)-4,4-difluoro-1 -(2-hydroxyhexanoyl)-N-methylpyrrolidine-2-carboxamide
- N-(1-((4-(3,5-difluorobenzamido)-1-(hydroxyamino)-1-oxobutan-2-yl)amino)-3 -methyl-1-oxopentan-2-yl)-4,4-difluoro-1 -(3-hydroxyhexanoyl)-N-methylpyrrolidine-2-carboxamide

- N-(1-((4-(3,5-difluorobenzamido)-1-(hydroxyamino)-1-oxobutan-2-yl)amino)-3 -methyl-1-oxopentan-2-yl)-4,4-difluoro-1 -(4-hydroxyhexanoyl)-N-methylpyrrolidine-2-carboxamide
- N-(1-((4-(3,5-difluorobenzamido)-1-(hydroxyamino)-1-oxobutan-2-yl)amino)-3-methyl-1-oxopentan-2-yl)-4,4-difluoro-1 -(5-hydroxyhexanoyl)-N-methylpyrrolidine-2-carboxamide
- N-(1-((4-(3,5-difluorobenzamido)-1-(hydroxyamino)-1-oxobutan-2-yl)amino)-3 -methyl-1-oxopentan-2-yl)-4,4-difluoro-1 -(6-hydroxyhexanoyl)-N-methylpyrrolidine-2-carboxamide

or a pharmaceutically acceptable salt or stereoisomer thereof.

In one embodiment of the present invention, the compounds of the invention have a specific stereochemical configuration in four of the chiral carbon atoms of formula (I), i.e. they are represented by formula (II) depicted below:

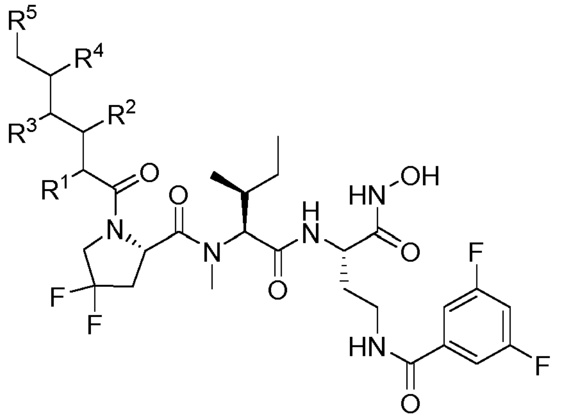

(II)

wherein each one of $R^1$, $R^2$, $R^3$, $R^4$ and $R^5$ is as previously defined or a pharmaceutically acceptable salt or stereoisomer thereof.

In one embodiment of the present invention the group $R^1$ in the compounds of formula (II) is a hydroxyl group and $R^2$, $R^3$, $R^4$ and $R^5$ are selected from the group consisting of hydrogen atoms and hydroxyl groups.

In one embodiment of the present invention the group $R^2$ in the compounds of formula (II) is a hydroxyl group and $R^2$, $R^3$, $R^4$ and $R^5$ are selected from the group consisting of hydrogen atoms and hydroxyl groups.

In one embodiment of the present invention the group $R^5$ in the compounds of formula (II) is a hydroxyl group and $R^1$, $R^2$, $R^3$ and $R^4$ are selected from the group consisting of hydrogen atoms and hydroxyl groups.

In one embodiment of the present invention the group $R^3$ in the compounds of formula (II) is a hydroxyl group and $R^1$, $R^2$, $R^4$ and $R^5$ are selected from the group consisting of hydrogen atoms and hydroxyl groups.

In one embodiment of the present invention the group $R^4$ in the compounds of formula (II) is a hydroxyl group and $R^1$, $R^2$, $R^3$ and $R^5$ are selected from the group consisting of hydrogen atoms and hydroxyl groups.

In one embodiment of the present invention in the compounds of formula (II) only one of $R^1$, $R^2$, $R^3$, $R^4$ and $R^5$ is a hydroxyl group and the rest are hydrogen atoms.

In one embodiment of the present invention the group $R^1$ in the compounds of formula (II) is a hydroxyl group and $R^2$, $R^3$, $R^4$ and $R^5$ are hydrogen atoms.

In one embodiment of the present invention the group $R^2$ in the compounds of formula (II) is a hydroxyl group and $R^2$, $R^3$, $R^4$ and $R^5$ are hydrogen atoms.

In one embodiment of the present invention the group $R^5$ in the compounds of formula (II) is a hydroxyl group and $R^1$, $R^2$, $R^3$ and $R^4$ are hydrogen atoms.

In one embodiment of the present invention the group $R^3$ in the compounds of formula (II) is a hydroxyl group and $R^1$, $R^2$, $R^4$ and $R^5$ are hydrogen atoms.

In one embodiment of the present invention the group $R^4$ in the compounds of formula (II) is a hydroxyl group and $R^1$, $R^2$, $R^3$ and $R^5$ are hydrogen atoms.

In one embodiment of the present invention the groups $R^1$, $R^2$ and $R^5$ are selected from hydroxyl group and hydrogen atom, with the proviso that at least one, and preferably only one of the groups $R^1$, $R^2$ and $R^5$ in the compounds of formula (II) is a hydroxyl group, and the groups $R^3$ and $R^4$ are hydrogen atoms.

In one embodiment, the compound of the invention is selected from the group consisting of:

- (2S)-N-((2S,3 S)-1-(((S)-4-(3,5-difluorobenzamido)-1-(hydroxyamino)-1-oxobutan-2-yl)amino)-3-methyl-1-oxopentan-2-yl)-4,4-difluoro- 1 -(2-hydroxyhexanoyl)-N-methylpyrrolidine-2-carboxamide

- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro- 1 -(3-hydroxyhexanoyl)-N-methylpyrrolidine-2-carboxamide
- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro- 1 -(4-hydroxyhexanoyl)-N-methylpyrrolidine-2-carboxamide
- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro- 1 -(5-hydroxyhexanoyl)-N-methylpyrrolidine-2-carboxamide
- (S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro- 1 -(6-hydroxyhexanoyl)-N-methylpyrrolidine-2-carboxamide

or a pharmaceutically acceptable salt thereof.

The compounds of formula (I) defined above (and the compounds of formula (II) defined above, which are a specific subgroup of the compounds of formula (I)) can be obtained by available synthetic procedures as illustrated by the following general scheme:

The 2-cholortrityl chloride resin (III) may be used as a polymeric support for the synthesis of the compounds of the invention. The resin may be placed in a syringe fitted with a polyethylene porous dish (resin vessel) and may be swelled, for example, by washes with appropriate organic solvents, such as dichloromethane (DCM) and dimethylformamide (DMF).

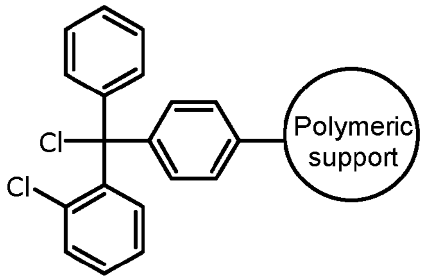

(III)

Following the swelling of the polymeric support, the linker Fmoc-NH-OH may be attached to the resin using an amide base, such as *N,N*-Diisopropylethylamine (DIEA), in an appropriate organic solvent, such as DCM. The mixture may be intermittently stirred during 24h. After which, without filtering the mixture, anhydrous methanol may be added to cap the remaining unreacted sites of the resin. After filtration and washing, Fmoc may be removed to yield product of formula (IV), for example, by a treatment with an amine base solution, such as piperidine in DMF and/or a mixture of piperidine/DBU/toluene/DMF. Filtrates and washings may be collected in a volumetric flask to quantify by UV measurements the achieved loading of the resin once the linker is anchored to the polymeric support. Fmoc removal may be also assessed using the Kaiser test.

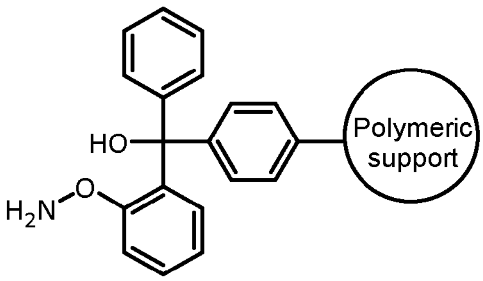

(IV)

Nα-Fmoc-Nγ-Alloc-2,4-diaminobutyric acid (Fmoc-Dab(Alloc)-OH) may be attached to the polymeric support of formula (IV) to yield the product of formula (V) using an coupling agent, such as *N,N'*-diisopropylcarbodiimide (DIC), in the presence or in the absence of an additive, such as ethyl cyano(hydroxyimino)acetate (Oxyma pure) in an appropriate organic solvent, such as DMF. The mixture may be stirred during the total reaction time, which may be of 1 hour. The extent of the coupling may be monitored using the Kaiser test and re-coupling may be done under the same conditions if required.

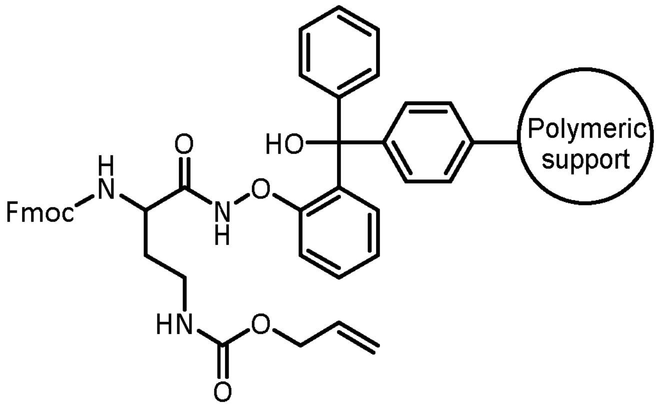

(V)

The Fmoc protecting group from the compound of formula (V) may be removed using an amine base solution, such as a piperidine in DMF and/or a mixture of piperidine/DBU/toluene/DMF to yield compound of formula (VI).

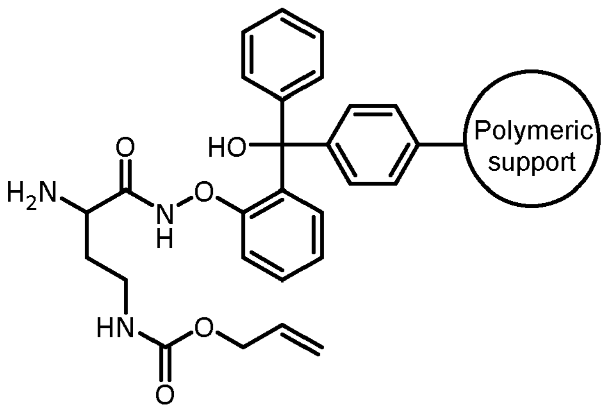

(VI)

Nα-Fmoc-methyl isoleucine (Fmoc-NMeIle-OH) may be attached to the compound of formula (VI) to yield the product of formula (VII) using an activating agent, such as DIC, in the presence or in the absence of an additive such as Oxyma pure, in an appropriate organic solvent, such as DMF. The mixture may be stirred during the total reaction time, for example 1 hour. The extent of the coupling may be monitored using the Kaiser test and re-coupling may be done under the same conditions if required.

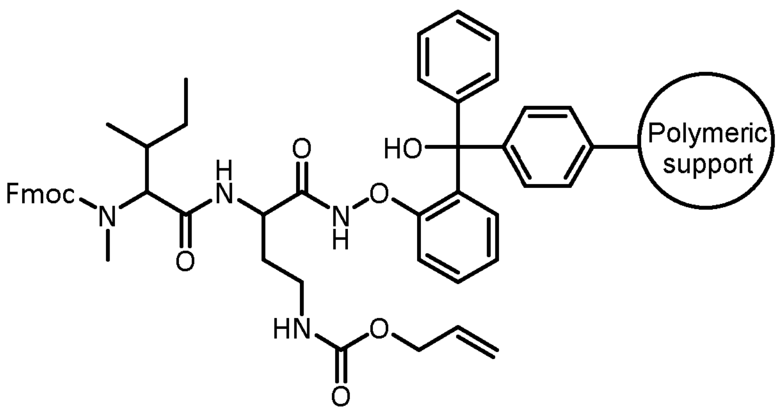

(VII)

The Fmoc protecting group from the compound of formula (VII) may be removed using an amine base solution,

such as a piperidine in DMF and/or a mixture of piperidine/DBU/toluene/DMF to yield compound of formula (VIII).

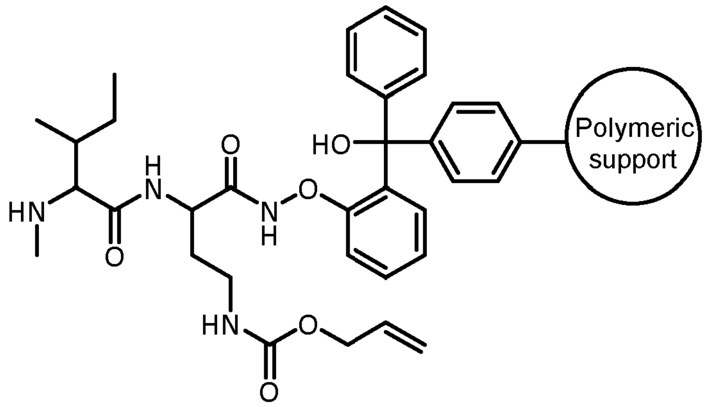

(VIII)

Nα-Fmoc-4,4-difluoro-proline (Fmoc-2,2F-Pro-OH) may be attached to the compound of formula (VIII) to yield the product of formula (IX) using an activating agent, such as PyBOP, in the presence or in the absence of an additive, such as HOAt, and an amide base, such as DIEA, in an appropriate organic solvent, such as DMF. The mixture may be stirred during the total reaction time, for example 1 hour. The extent of the coupling may be monitored using the Kaiser test and re-coupling may be done under the same conditions if required.

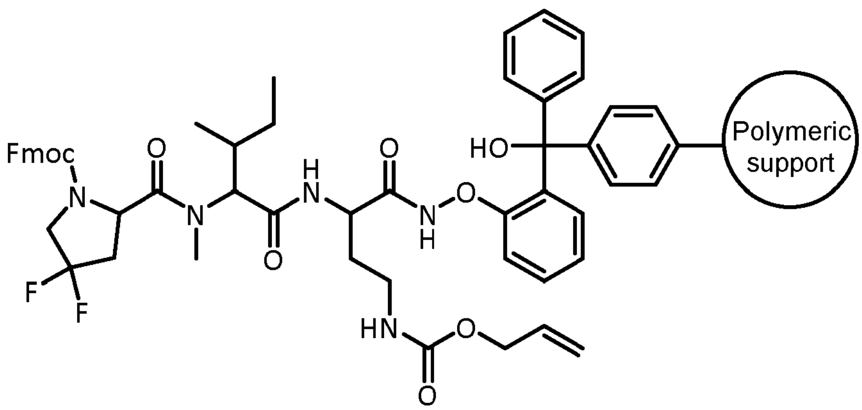

(IX)

The Fmoc protecting group from the compound of formula (IX) may be removed by an amine base solution, such as a piperidine in DMF and/or a mixture of piperidine/DBU/toluene/DMF, to yield compound of formula (X).

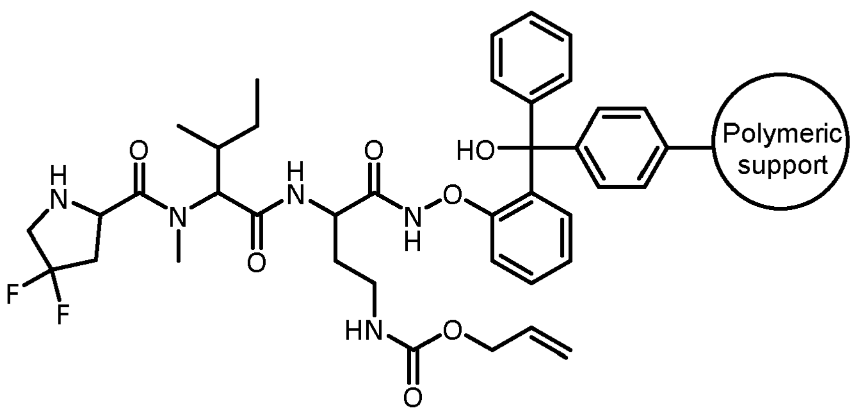

(X)

Once the compound of formula (X) has been obtained, the hexanoic acid derivative of formula (XI)

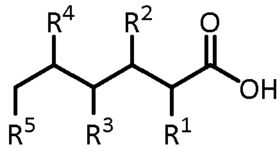

(XI),

wherein $R^1$, $R^2$, $R^3$, $R^4$ and $R^5$ are as hereinabove defined, may be coupled to yield the product of formula (XII) using an activating agent, such as PyBOP, in the presence or in the absence of an additive, such as HOAt, and an amine base, such as DIEA, in an appropriate organic solvent, such as DMF. The mixture may be stirred during the total reaction time, for example 1 to 2 hours. The extent of the coupling may be monitored using the Kaiser test and recoupling may be done under the same conditions if required.

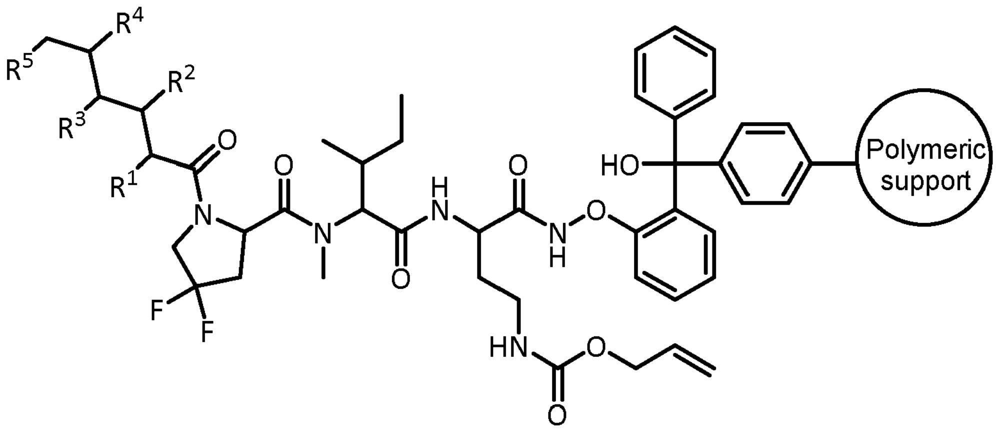

(XII)

The Alloc protecting group from the compound of formula (XII) may be removed to yield a compound of formula (XIII), for example by suspending the compound of formula (XII) in an organic solvent, such as DCM, and adding phenylsilane to it, while $N_2$ is bubbled through the suspension. Then, tetrakis(triphenylphosphine)palladium(0) may be added, and the $N_2$-bubbling maintained for 5 minutes. After that, the reaction vessel may be sealed and shaken for 15 min. After this time, the reaction may be filtered, and the resin washed thoroughly. The same treatment may be repeated two more times. After the last treatment, the resin may be washed thoroughly with DCM, methanol and DMF. The extent of the removal of the alloc protecting group may monitored using the Kaiser test.

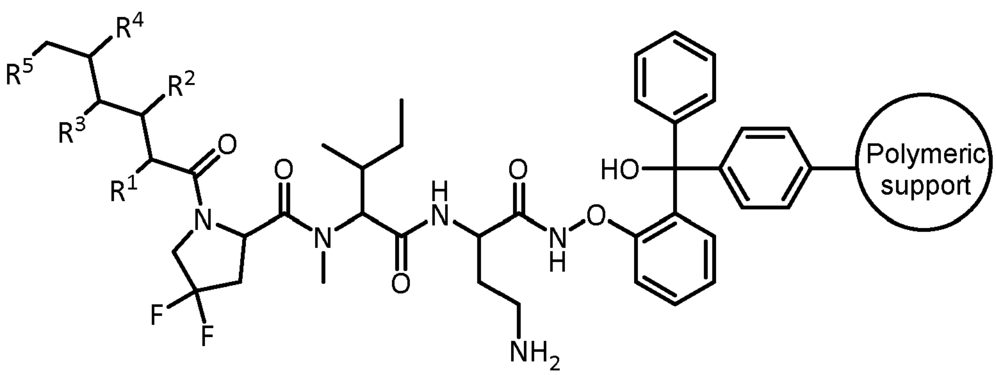

(XIII)

Subsequently, 3,5-difluorobenzoic acid may be coupled to the amino group of the side chain of the diaminobutiric acid moiety of compound of formula (XIII), for example using an activating agent, such as *N,N'*-Diisopropylcarbodiimide (DIPCDI), in the presence or in the absence of an additive, such as HOAt in DMF. The mixture may be intermittently stirred

manually for 1 hour. Then the reaction mixture may be filtered off and the resin may be washed thoroughly with DMF, MeOH and DCM. The extent of the coupling reaction may be monitored using the Kaiser test. A recoupling step of the 3,5-difluorobenzoic acid may be carried out when the colorimetric test shows that the coupling reaction is not completely achieved. This step yields compound of formula (XIV).

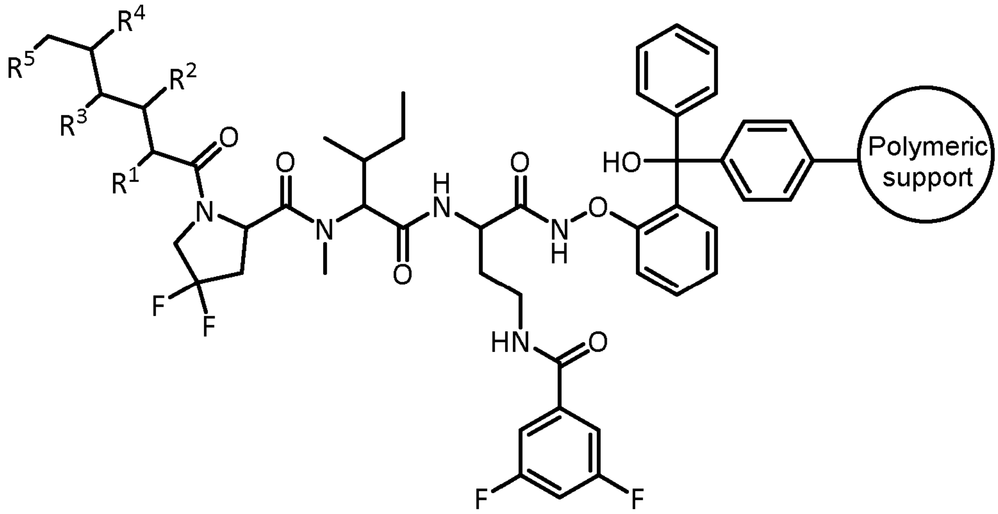

(XIV)

The compound of formula (XIV) may be cleaved from the resin, for example by adding an acid, such as trifluoroacetic acid (TFA) in DCM (TFA/DCM 5:95). The mixture may be stirred at room temperature for 15 min. After this time, the resin may be filtered off and washed several times with TFA/DCM (5:95) and then DCM. After that the filtrates obtained may be pooled and the solvent may be evaporated under vacuum to yield compound of formula (I).

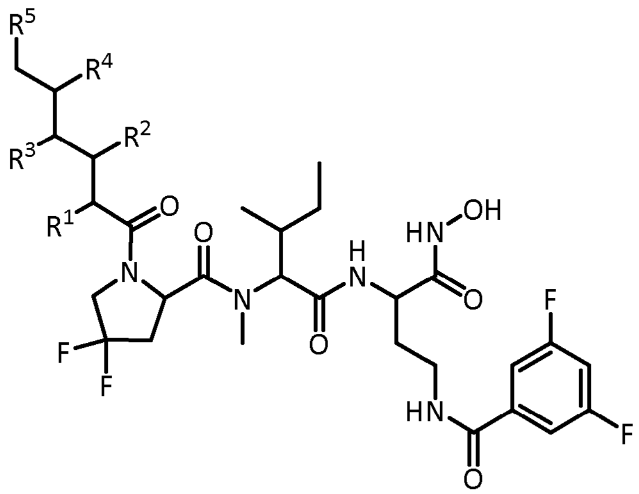

(I)

wherein $R^1$, $R^2$, $R^3$, $R^4$ and $R^5$ are as previously defined.

A second aspect of the invention refers to pharmaceutical compositions comprising a compound of formula (I) as defined in the first aspect and a pharmaceutically acceptable carrier. In an embodiment the pharmaceutical compositions may contain more than one carrier, in particular when they comprise water as one of the carriers.

Further, the term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the active ingredient is

administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

Examples of pharmaceutical compositions include any solid (tablets, pills, capsules, granules etc.) or liquid (solutions, suspensions or emulsions) composition for oral, topical or parenteral administration.

The pharmaceutical compositions may be in oral form, either solid or liquid. Suitable dose forms for oral administration may be tablets, capsules, syrups or solutions and may contain conventional excipients known in the art such as binding agents, for example syrup, acacia gum, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tableting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

A third aspect of this invention refers to a compound of formula (I) as defined in the first aspect, or a pharmaceutical composition as defined in the second aspect, for use as a medicament, particularly for the prevention and/or treatment of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

Another aspect of this invention refers to a method for the treatment or prophylaxis of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis in a mammal wherein a therapeutic amount of a compound of formula (I) as defined in the first aspect, or a pharmaceutical composition as defined in the second aspect, is administered to a patient in need of said treatment.

Another aspect of this invention refers to the use of a compound of formula (I) as defined in the first aspect, or a pharmaceutical composition as defined in the second aspect, for the preparation of a medicament, particularly for the prevention and/or treatment of a disease selected from the group consisting of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

The terms "treat", "treatment", or "treatment of" as used herein refer to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. It also refers to mitigating or decreasing at least one clinical symptom and/or inhibition or delay in the progression of the condition and/or prevention or delay of the onset of a disease or illness.

The terms "prevention", "preventing" or "prevent" as used herein refer to avoiding the appearance of a certain disease or disorder. The prevention can be complete (e.g., the total absence of a disease). The prevention can also be partial, such that for example the occurrence of a disease in a subject is less than that which would have occurred without the administration of the combination or composition of the present invention. Prevention also refers to reduced susceptibility to a clinical condition. The prevention also includes reducing the risk of suffering the disease.

The subject to which the compounds and pharmaceutical compositions described herein are administered is a human or animal; preferably subjects are mammals, more preferably humans.

By a "therapeutic amount" of a drug or pharmacologically active agent is meant a nontoxic but sufficient amount of the drug or agent to provide the desired effect. The amount that is "therapeutic" will vary from subject to subject, depending on the age and general condition of the individual, the particular active agent or agents, and the like.

The following examples represent specific embodiments of the present invention. They do not intend to limit in any way the scope of the invention defined in the present description.

## EXAMPLES

### 1. Synthesis of compounds of formula (I)

**Example 1: (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1-(2-hydroxyhexanoyl)-N-methylpyrrolidine-2-carboxamide**

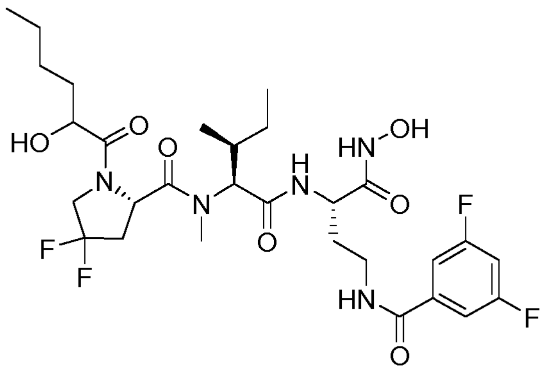

1.5 eq of commercially available Fmoc-NH-OH and DIEA (10 eq) are added to the 2-chlorotrityl resin in 2 mL DCM. The mixture is intermittently stirred manually during 24h. After that, 0.5 mL/g of MeOH are added to the reaction mixture to cap the remaining reactive points of the resin. After 15 minutes, the solution is filtered off and the resin is washed thoroughly with DCM, DMF and MeOH. Fmoc removal is achieved by treating the resin with 20% piperidine in DMF (1 x 5', 1 x 10' and 1 x 15'). For the coupling of Nα-Fmoc-Nγ-alloc-L-2,4-diaminobutyric acid (Fmoc-L-Dab(alloc)-OH), 3 eq of the amino acid, 3 eq of the coupling agent DIC and 3 eq of oxyma pure are dissolved in a small amount of DMF and premixed for 2 minutes. The resulting mixture is added to the resin and the reaction is allowed to proceed for 60 minutes. To extent of the reaction is monitored using the Kaiser test. The Fmoc group is then removed by treatments with 20% piperidine in DMF (1 x 5', 1 x 1 O' and 1 x 15'). After that, Fmoc-N-methyl-L-isoleucine (Fmoc-NMe-L-Ile-OH) moiety is attached, for that purpose 3 eq of the amino acid, 3 eq of the coupling agent DIC and 3 eq of oxyma pure are dissolved in a small amount of DMF and premixed for 2 minutes. The resulting mixture is added to the resin and the reaction is allowed to proceed for 60 minutes. The extent of the reaction is monitored using the Kaiser test. The Fmoc group is then removed by treatments with 20% piperidine in DMF (1 x 5', 1 x 1 O' and 1 x 15'). After that, the Fmoc-L-( 4,4-difluoro)Pro-OH moiety is attached, for that purpose 4 eq of the amino acid, 4 eq of the coupling agent PyBOP, 12eq of the additive HOAt and 12 eq of DIEA dissolved in a small amount of DMF and premixed for 2 minutes. After which, the mixture is added to the resin and the reaction is allowed to react for 90 minutes. The extent of the reaction is monitored using the Kaiser test. The Fmoc group is then removed by treatments with 20% piperidine in DMF (1 x 5', 1 x 10' and 1 x 15') and additional treatment with a mixture of piperidine/DBU/toluene/DMF (5:5:20:70) (1 x 5'). 2-hydroxyhexanoic acid is coupled to the Pro moiety by adding to the resin 4 eq of the acid, 4 eq of the coupling regent PyBOP, 12eq of the additive HOAt and 12 eq of DIEA. The reaction is stirred manually intermittently for 60 minutes. Then the reaction is filtered off and the resin is rinsed thoroughly with DMF and DCM. The extent of the reaction is monitored using the chloranil test. For the removal of the Alloc group, 10 eq of phenylsilane in DCM are added to the resin while $N_2$ is bubbled through the mixture. Then, 0.1 eq of $Pd(PPh_3)_4$ are added maintaining the $N_2$ bubbling while mixing everything well. Then the reaction vessel is sealed and shaken for 15 minutes. After this time, the reaction is filtered and the resin washed thoroughly. The same treatment is repeated two more times. After the last treatment, the resin is washed thoroughly with DCM, MeOH and DMF. For the coupling of the 3,5-difluorobenzoic acid on the side chain of the Dab moiety, 5 eq of said acid, 5 eq of DIPCDI, 5 eq of HOAt and 0.5 eq of DMAP in DMF are added to the resin. The reaction is allowed to react. for 60 minutes. After this time, the resin is washed with DMF and DCM and the extent of the reaction is monitored the Kaiser test. For the cleavage of the peptide, the resin is washed several times with DCM and dried by suction. The peptide is cleaved from the resin by adding a solution of DCM/TFA (95:5), the mixture is allowed to react for 15 min. Then the reaction mixture is filtered, and the resin rinsed with DCM. This cleavage procedure is repeated twice. All the filtrates are pooled, and the solvent is evaporated under vacuum, yielding example 1. The compound is purified using reverse-phase chromatography.

$^1$H-NMR (DMSO-d6, 400MHz) δ(ppm): 8.71 (1H, t), 8.50 (1H, s), 7.48 (3H, m), 4.99 (1H, dt), 4.37 (1H, m), 4.19

(1H, m), 3.72 (7H, m), 3.02 (2H, d), 2.54 (1H, s), 1.93 (3H, s), 1.80 (2H, s), 1.28 (6H, m), 0.89 (3H, s), 0.83 (7H, dq).

**Example 2: (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1-(3-hydroxyhexanoyl)-N-methylpyrrolidine-2-carboxamide**

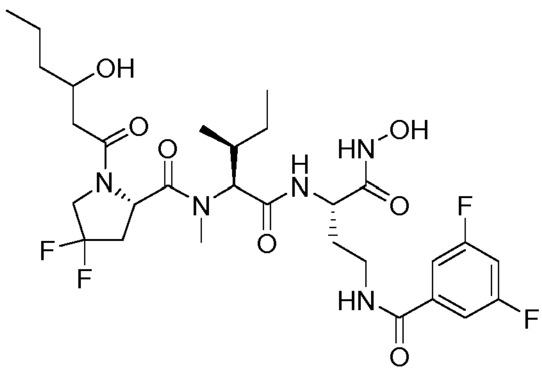

1.5 eq of commercially available Fmoc-NH-OH and DIEA (10 eq) are added to the 2-chlorotrityl resin in 2 mL DCM. The mixture is intermittently stirred manually during 24h. After that, 0.5 mL/g of MeOH are added to the reaction mixture to cap the remaining reactive points of the resin. After 15 minutes, the solution is filtered off and the resin is washed thoroughly with DCM, DMF and MeOH. Fmoc removal is achieved by treating the resin with 20% piperidine in DMF (1 x 5', 1 x 10' and 1 x 15'). For the coupling of Nα-Fmoc-Nγ-alloc-L-2,4-diaminobutyric acid (Fmoc-L-Dab(alloc)-OH), 3 eq of the amino acid, 3 eq of the coupling agent DIC and 3 eq of oxyma pure are dissolved in a small amount of DMF and premixed for 2 minutes. The resulting mixture is added to the resin and the reaction is allowed to proceed for 60 minutes. To extent of the reaction is monitored using the Kaiser test. The Fmoc group is then removed by treatments with 20% piperidine in DMF (1 x 5', 1 x 1 O' and 1 x 15'). After that, Fmoc-N-methyl-L-isoleucine (Fmoc-NMe-L-Ile-OH) moiety is attached, for that purpose 3 eq of the amino acid, 3 eq of the coupling agent DIC and 3 eq of oxyma pure are dissolved in a small amount of DMF and premixed for 2 minutes. The resulting mixture is added to the resin and the reaction is allowed to proceed for 60 minutes. The extent of the reaction is monitored using the Kaiser test. The Fmoc group is then removed by treatments with 20% piperidine in DMF (1 x 5', 1 x 1 O' and 1 x 15'). After that, the Fmoc-L-( 4,4-difluoro)Pro-OH moiety is attached, for that purpose 4 eq of the amino acid, 4 eq of the coupling agent PyBOP, 12eq of the additive HOAt and 12 eq of DIEA dissolved in a small amount of DMF and premixed for 2 minutes. After which, the mixture is added to the resin and the reaction is allowed to react for 90 minutes. The extent of the reaction is monitored using the Kaiser test. The Fmoc group is then removed by treatments with 20% piperidine in DMF (1 x 5', 1 x 10' and 1 x 15') and additional treatment with a mixture of piperidine/DBU/toluene/DMF (5:5:20:70) (1 x 5'). 3-hydroxyhexanoic acid is coupled to the Pro moiety by adding to the resin 4 eq of the acid, 4 eq of the coupling regent PyBOP, 12eq of the additive HOAt and 12 eq of DIEA. The reaction is stirred manually intermittently for 60 minutes. Then the reaction is filtered off and the resin is rinsed thoroughly with DMF and DCM. The extent of the reaction is monitored using the chloranil test. For the removal of the Alloc group, 10 eq of phenylsilane in DCM are added to the resin while $N_2$ is bubbled through the mixture. Then, 0.1 eq of $Pd(PPh_3)_4$ are added maintaining the $N_2$ bubbling while mixing everything well. Then the reaction vessel is sealed and shaken for 15 minutes. After this time, the reaction is filtered and the resin washed thoroughly. The same treatment is repeated two more times. After the last treatment, the resin is washed thoroughly with DCM, MeOH and DMF. For the coupling of the 3,5-difluorobenzoic acid on the side chain of the Dab moiety, 5 eq of said acid, 5 eq of DIPCDI, 5 eq of HOAt and 0.5 eq of DMAP in DMF are added to the resin. The reaction is allowed to react. for 60 minutes. After this time, the resin is washed with DMF and DCM and the extent of the reaction is monitored the Kaiser test. For the cleavage of the peptide, the resin is washed several times with DCM and dried by suction. The peptide is cleaved from the resin by adding a solution of DCM/TFA (95:5), the mixture is allowed to react for 15 min. Then the reaction mixture is filtered, and the resin rinsed with DCM. This cleavage procedure is repeated twice. All the filtrates are pooled, and the solvent is evaporated under vacuum, yielding example 2. The compound is purified using reverse-phase chromatography.

$^{1}$H-NMR (DMSO-d6, 400MHz) δ(ppm): 8.69 (1H, d), 7.99 (1H, dd), 7.61 (1H, m), 7.47 (4H, m), 5.26 (1H, m), 4.63 (1H, m), 4.27 (3H, m), 3.81 (1H, s), 3.16 (4H, s), 2.8 (3H,d), 2.40 (1H, m), 1.92 (3H, s), 1.79 (1H, s), 1.30 (6H, m), 0.82 (6H, dddd).

**Example 3: (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1-(6-hydroxyhexanoyl)-N-methylpyrrolidine-2-carboxamide**

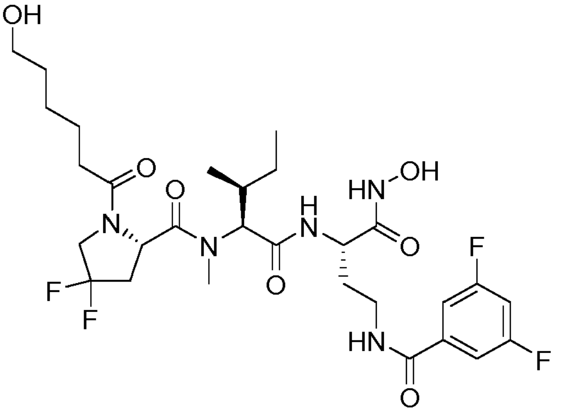

1.5 eq of commercially available Fmoc-NH-OH and DIEA (10 eq) are added to the 2-chlorotrityl resin in 2 mL DCM. The mixture is intermittently stirred manually during 24h. After that, 0.5 mL/g of MeOH are added to the reaction mixture to cap the remaining reactive points of the resin. After 15 minutes, the solution is filtered off and the resin is washed thoroughly with DCM, DMF and MeOH. Fmoc removal is achieved by treating the resin with 20% piperidine in DMF (1 x 5', 1 x 10' and 1 x 15'). For the coupling of Nα-Fmoc-Nγ-alloc-L-2,4-diaminobutyric acid (Fmoc-L-Dab(alloc)-OH), 3 eq of the amino acid, 3 eq of the coupling agent DIC and 3 eq of oxyma pure are dissolved in a small amount of DMF and premixed for 2 minutes. The resulting mixture is added to the resin and the reaction is allowed to proceed for 60 minutes. To extent of the reaction is monitored using the Kaiser test. The Fmoc group is then removed by treatments with 20% piperidine in DMF (1 x 5', 1 x 1 O' and 1 x 15'). After that, Fmoc-N-methyl-L-isoleucine (Fmoc-NMe-L-Ile-OH) moiety is attached, for that purpose 3 eq of the amino acid, 3 eq of the coupling agent DIC and 3 eq of oxyma pure are dissolved in a small amount of DMF and premixed for 2 minutes. The resulting mixture is added to the resin and the reaction is allowed to proceed for 60 minutes. The extent of the reaction is monitored using the Kaiser test. The Fmoc group is then removed by treatments with 20% piperidine in DMF (1 x 5', 1 x 1 O' and 1 x 15'). After that, the Fmoc-L-( 4,4-difluoro)Pro-OH moiety is attached, for that purpose 4 eq of the amino acid, 4 eq of the coupling agent PyBOP, 12eq of the additive HOAt and 12 eq of DIEA dissolved in a small amount of DMF and premixed for 2 minutes. After which, the mixture is added to the resin and the reaction is allowed to react for 90 minutes. The extent of the reaction is monitored using the Kaiser test. The Fmoc group is then removed by treatments with 20% piperidine in DMF (1 x 5', 1 x 10' and 1 x 15') and additional treatment with a mixture of piperidine/DBU/toluene/DMF (5:5:20:70) (1 x 5'). 6-hydroxyhexanoic acid is coupled to the Pro moiety by adding to the resin 4 eq of the acid, 4 eq of the coupling regent PyBOP, 12eq of the additive HOAt and 12 eq of DIEA. The reaction is stirred manually intermittently for 60 minutes. Then the reaction is filtered off and the resin is rinsed thoroughly with DMF and DCM. The extent of the reaction is monitored using the chloranil test. For the removal of the Alloc group, 10 eq of phenylsilane in DCM are added to the resin while $N_2$ is bubbled through the mixture. Then, 0.1 eq of $Pd(PPh_3)_4$ are added maintaining the $N_2$ bubbling while mixing everything well. Then the reaction vessel is sealed and shaken for 15 minutes. After this time, the reaction is filtered and the resin washed thoroughly. The same treatment is repeated two more times. After the last treatment, the resin is washed thoroughly with DCM, MeOH and DMF. For the coupling of the 3,5-difluorobenzoic acid on the side chain of the Dab moiety, 5 eq of said acid, 5 eq of DIPCDI, 5 eq of HOAt and 0.5 eq of DMAP in DMF are added to the resin. The reaction is allowed to react. for 60 minutes. After this time, the resin is washed with DMF and DCM and the extent of the reaction is monitored the Kaiser test. For the cleavage of the peptide, the resin is washed several times with DCM and dried by suction. The peptide is cleaved from the resin by adding a solution of DCM/TFA (95:5), the mixture is allowed to react for 15 min. Then the reaction mixture is filtered, and the resin rinsed with DCM. This cleavage procedure is repeated twice. All the filtrates are pooled, and the solvent is evaporated under vacuum, yielding example 3. The compound is purified using reverse-phase chromatography.

$^1$H-NMR (DMSO-d6, 400MHz) δ(ppm): 8.71 (1H, t), 8.51 (1H, m), 7.97 (1H, m), 7.61 (1H, m), 7.47 (3H, m), 5.24 (1H, t), 4.94 (1H, m), 4.63 (1H, m), 4.25 (1H, m), 3.95 (1H, dt), 3.37 (3H, ddd), 3.27 (1H, m), 3.18 (1H, m), 2.95 (1H, dd), 2.54 (4H, s), 2.25 (1H, m), 1.93 (2, m), 1.79 (1H, m), 1.47 (2H, m), 1.39 (1H, m), 1.28 (2H, m), 0.83 (10H, m).

**2. Inhibitory effect on matrix MMP-2 and MMP-9**

General considerations: all the experiments were done in 1X reaction buffer, which was obtained from a 10X

reaction buffer solution. The content of the 10X reaction buffer was: 50 mL of 0.5 M Tris-HCl, 1.5 M NaCl, 50 mM $CaCl_2$ and 2 mM sodium azide at pH 7.6. To obtain the 1X reaction buffer 4 mL of the 10X reaction buffer were diluted in 36 mL of $H_2O$.

**MMP-2 inhibition assay:**

Recombinant MMP-2 expressed in Chinese hamster ovary (CHO) cells was obtained from Merck (catalog number PF023-5UG). DQ gelatin from pig skin fluorescein conjugated (MMP-2 substrate) was obtained from Thermo-Fisher (catalog number E12055).

Preparation of the MMP-2 for the activity assay: MMP-2 was provided as a stock solution (0.1 mg/mL). The enzyme was diluted in 1X reaction buffer to a final concentration of 5 μg/mL.

Preparation of the DQ gelatin (substrate) solution for the activity assay: the solid form of the substrate was dissolved in water in order to obtain a stock solution of 1 mg/mL.

Procedure: The enzymatic assays were performed in 96-well microtiter plate, which allowed simultaneous monitoring of multiple reactions. For each reaction, 86.3 μL of 1X reaction buffer (pH 7.6), 1.7 μL of MMP-2 (final concentration 85 ng/mL) and 2 μL of the corresponding new compound were added to each well. A stock solution of new compound was prepared in DMSO (100 mM), and dilutions were prepared from this stock solution with DMSO. Finally, to the mixture contained in each well 10 μL of the substrate were added (final concentration 50 μg/mL). The reaction was incubated for 2 hours at room temperature in an orbital shaker (100 rpm).

The inhibitory activity of the new compound was measured fluorimetrically. The excitation and emission wavelengths were 483 and 525 nm, respectively.

**MMP-9 inhibition assay:**

Recombinant MMP-9 expressed was obtained from Merck (catalog number PF140-5UG). DQ gelatin from pig skin fluorescein conjugated (MMP-9 substrate) was obtained from ThermoFisher (catalog number E12055).

Preparation of the MMP-9 for the activity assay: MMP-9 was provided as a stock solution (0.1 mg/mL). The enzyme was diluted in 1X reaction buffer to a final concentration of 5 μg/mL.

Preparation of the DQ gelatin (substrate) solution for the activity assay: the solid form of the substrate was dissolved in water in order to obtain a stock solution of 1 mg/mL.

Procedure: The enzymatic assays were performed in 96-well microtiter plate, which allowed simultaneous monitoring of multiple reactions. For each reaction, 84.6 μL of 1X reaction buffer (pH 7.6), 3.4 μL of MMP-9 (final concentration 85 ng/mL) and 2 μL of the corresponding new compound were added to each well. A stock solution of new compound was prepared in DMSO (100 mM), and dilutions were prepared from this stock solution with DMSO. Finally, to the mixture contained in each well 10 μL of the substrate were added (final concentration 50 μg/mL). The reaction was incubated for 4 hours at room temperature in an orbital shaker (100 rpm).

The inhibitory activity of the new compound was measured fluorimetrically. The excitation and emission wavelengths were 483 and 525 nm, respectively.

Inhibitory activity at several concentration points (ranging from 2 nM to 40 μM MMP) were measured for each compound. The inhibitory activity on MMP-2 and MMP-9 was calculated according to eq 1. For each new compound, the fluorescence in the presence (a) and in the absence (b) of the corresponding MMP was measured. The maximum fluorescence (0% inhibitory activity) was obtained from a sample of the corresponding MMP in the absence of inhibitory compounds. To estimate the inhibitory potency of the novel compounds, activities were plotted against the log concentration of the compound, adjusting to a sigmoid curve using GraphPad Prism software, and the $IC_{50}$ value, defined as the concentration of compound required to inhibit 50% of the corresponding MMP activity, was determined from resulting curve.

$$Inhibitory\ activity\ (\%) = \left[1 - \left(\frac{a-b}{c-d}\right)\right] x\ 100\ (Equation\ 1)$$

wherein:

a corresponds to fluorescence intensity in the presence of substrate + test compound + MMP
b corresponds to the fluorescence intensity in the presence of substrate + tested compound
c corresponds to the fluorescence intensity in the presence of substrate + MMP
d corresponds to the fluorescence intensity of the presence of substrate

The results are summarized in table 1.

**Table 1:** Inhibition of MMP-2 and MMP-9

| Compound (Example n°) | MMP-9 $IC_{50}$ (nM) | MMP-2 $IC_{50}$ (nM) |
|---|---|---|
| **1** | 2070 | - |
| **2** | 16.2 | 15.4 |
| **3** | 267 | 104 |

## 3. Stability and potential drug-drug interaction (DDI) liability risk of the compounds

The stability and DDI liability risk of the compounds of the invention was determined and compared to that of prior art MMP-9 and MMP-2 inhibitors from example 7 (i.e. (2S)-N-[(1S)-1-[[(1S)-3-[(3,5-difluorobenzoyl)amino]-1-(hydroxycarbamoyl) propyl]carbamoyl]-2-methyl-butyl]-4,4-difluoro-1-hexanoyl-N-methyl-pyrrolidine-2-carboxamide) and example 16 (i.e. (2S,4R)-N-[(1S)-1-[[(1S)-3-benzamido-1-(hydroxycarbamoyl)propyl]carbamoyl]-2-methyl-butyl]-4-fluoro-N-methyl-1-hexanoyl-pyrrolidine-2-carboxamide) of WO2017085034A1.

**Stability assay in human hepatocytes**

Human hepatocytes were obtained from Bioreclamation IVT (catalog number X008001). Cryopreserved cells were thawed in Williams' Medium E, isolated, and suspended in Incubation Medium (Williams' Medium E without phenol red containing L-Glutamine and HEPES).

Procedure: the stability assay was performed in 96-well microtiter plate, which allowed simultaneous monitoring of multiple reactions. For each reaction, 198 μL of a suspension containing $0.5\times10^6$ cells/mL was used. Plates were incubated at 37°C in a 95% humidified incubator at 5% $CO_2$ under constant shaking. In parallel, a stop solution (acetonitrile containing tolbutamide and an internal standard) was prepared. 125 μL of this stop solution was transferred to a set of pre-labeled 96-well plates and kept in an ice-cold bath.

To start the reactions, 2 μL of a DMSO solution containing the compound under evaluation (100 μM) was spike in each reaction well containing the cells in duplicate (final concentration 1 μM). The plate was kept in the incubator under shaking and at selected time points (0, 15, 30, 60, and 90 minutes), samples were mixed and then 25 μL of each sample at each time point was transferred to wells containing 125 μL of the ice-cold stop solution followed by mixing. Samples in the stop solution mixture were immediately vortexed on a plate shaker at 600 rpm for 10 minutes, then centrifuged at 3220 × g for 20 minutes at 4°C. After centrifugation, 80 μL/well of supernatant in the sample plates were transferred to another set of pre-labeled 96-well plates containing 240 μL of ultra-pure water in each well and were stored at 4°C until LC-MS/MS analysis.

The remaining percentages of test articles after incubation were calculated by the following equation:

$$\%\ Remaining = \left(\frac{Peak\ area\ ratio\ of\ analyte\ to\ internal\ standard\ at\ each\ time\ point}{Peak\ area\ ratio\ of\ analyte\ to\ internal\ standard\ at\ t\ =\ 0}\right) x\ 100$$

The equation of first-order kinetics was used to calculate $t_{1/2}$ and CLint:

$$Ct = C0 \cdot e^{-kt}$$

When

$$Ct = \frac{1}{2}C0:$$

$$t1/2 = \frac{ln2}{k} = \frac{0.693}{k}$$

$$CLint(hep) = \frac{K}{million\ cells\ per\ mL}$$

$$CLint(liver) = CLint(hep)\ x\ liver\ weight\ (g\ /kg\ body\ weight) x\ hepatocellularity$$

**Drug-drug interaction liability (CYP3A4 inhibition assay)**

Human liver microsomes were obtained from Corning (catalogue number 452117). Microsomes were taw on ice and a working solution in potassium phosphate buffer was prepared (final concentration 0.200 mg/mL).

Procedure: experiments were done in 48-well plates. 158 μL of the microsome working solution was added to the wells and the plate was prewarmed at 37°C for 10 minutes in water bath. In each well, 20 μL of 10 mM midazolam (substrate) solution in MeOH was added (an equivalent volume of potassium phosphate buffer was used for blanks) as well as 2 μL of the test compound under evaluation (5 nM to 50 μM final concentration). After that, 20 μL of a NAPDH solution (1 mM) was added to all incubation wells for activation. The mixture was incubated for 10 minutes at 37°C and the reaction was stopped afterwards by adding 400 μL of a cold acetonitrile to each well. The samples were centrifuged at 4000 rpm for 20 minutes to precipitate proteins. 200 μL of the resulting supernatant were mixed with 100 μL of HPLC water and shaken for 10 minutes. The concentration of midazolam for each sample was calculated by means of LC-MS/MS analysis. For data analysis, SigmaPlot was used to plot the percent of vehicle control versus the test substrate concentrations for non-linear regression analysis. $IC_{50}$ values were determined using 3- parameter logistic equations:

$$y = \frac{max}{1 + \left(\frac{X}{IC_{50}}\right)^{-hillslope}}$$

**Results**

• Stability in human hepatocytes

| *Compound* | *$T_{1/2}$ (min)* | *Clint (hep) (μL/min/$10^6$)* | *Clint (liver) (mL/min/kg)* |
|---|---|---|---|
| Example 1 | >216.8 | <6.4 | <17.8 |
| Example 2 | >216.8 | <6.4 | <17.8 |
| Example 3 | >216.8 | <6.4 | <17.8 |
| Comparative example - ex7 WO2017085034A1 | 84.4 | 16.4 | 45.6 |
| Comparative example - ex16 WO2017085034A1 | 141.1 | 9.8 | 27.3 |

Drug stability in hepatocytes is crucial because it directly impacts the drug's pharmacokinetics, efficacy, and safety profile. Drugs with low hepatocyte half-life or high intrinsic clearance pose challenges such as frequent dosing, reduced efficacy, increased first-pass effect, and variability in patient response.

Half-life (t1/2): a drug with a low t1/2 in hepatocytes is rapidly metabolized. This can lead to several clinical implications:

- Frequent dosing: the drug may require frequent dosing to maintain therapeutic levels, which can be inconvenient for patients and may reduce compliance.
- Reduced efficacy: Rapid metabolism can lead to lower plasma concentrations, potentially reducing the drug's efficacy.

Intrinsic clearance (Clint): high intrinsic clearance indicates that the drug is efficiently metabolized by the liver enzymes. This has several consequences:

- Short half-life: similar to low stability, high intrinsic clearance results in a short half-life, necessitating frequent dosing.
- Increased first-pass effect: drugs with high intrinsic clearance may undergo extensive first-pass metabolism when administered orally, significantly reducing their bioavailability.
- Dose Adjustments: Higher doses might be required to achieve therapeutic effects, but this must be balanced against the risk of toxicity.

New compounds (example 1-3) have a higher half-life and reduced Clint compared to previous state of the art reference compounds (comparative example 7 and 16 of WO2017085034A1). This surprising increased stability is achieved by adding an hydroxyl moiety in the hexanoic acid chain.

• DDI liability -CYP3A4 inhibition

| *Compound* | *IC50 (μM)* |
|---|---|
| Example 1 | 3.9 |
| Example 2 | 17.3 |
| Example 3 | 16.6 |
| Comparative example - ex7 WO2017085034A1 | 1.03 |
| Comparative example - ex16 WO2017085034A1 | 1.58 |

Inhibition of CYP3A4 is a significant risk since it impacts drug safety and efficacy. Careful consideration is necessary when developing new drugs or coadministering drugs that may inhibit this enzyme.

CYP3A4 is one of the most important enzymes in the cytochrome P450 family and is responsible for the metabolism of a large proportion of drugs. The risks on CYP3A4 inhibition relates to the following factors:

- DDIs: CYP3A4 metabolizes more than the 50% of all marketed drugs. Inhibition of this enzyme can lead to increased plasma concentrations of co-administered drugs that are CYP3A4 substrates, potentially causing toxicity or adverse effects.
- Altered pharmacokinetics: Inhibition of CYP3A4 can affect the pharmacokinetic profile of drugs, leading to prolonged half-life and increased exposure. This can result in overdose and serious side effects.
- Endogenous substrate metabolism: CYP3A4 also metabolizes endogenous substrates, including steroid hormones. Inhibition of CYP3A4 can disrupt the balance of these hormones, potentially leading to endocrine disorders.
- Variable patient response: There is significant interindividual variability in CYP3A4 activity due to genetic polymorphisms, age, sex, diet, and disease state. Inhibitors can exacerbate these variations, leading to unpredictable drug responses.
- Therapeutic failure: Some drugs require bioactivation by CYP3A4 to become therapeutically active. Inhibition of CYP3A4 can prevent the activation of such prodrugs, resulting in therapeutic failure.

New compounds (example 1-3) have a lower capacity to inhibit CYP3A4 compared to previous state of the art reference compounds (comparative example 7 and 16 of WO2017085034A1). The risk of potential DDI liability is surprisingly reduced by adding an hydroxyl moiety in the hexanoic acid chain.

## Claims

**1.** A compound of formula (I):

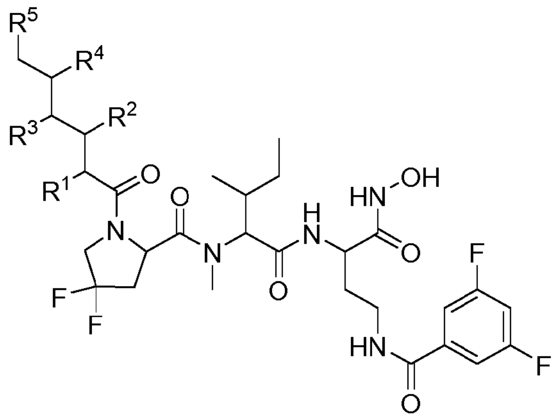

(I)

wherein

each one of $R^1$, $R^2$, $R^3$, $R^4$ and $R^5$ is independently selected from a hydrogen atom and a hydroxyl group, with the proviso that at least one of $R^1$, $R^2$, $R^3$, $R^4$ and $R^5$ is a hydroxyl group,
or a pharmaceutically acceptable salt thereof or stereoisomer thereof.

**2.** A compound according to claim 1, wherein $R^1$ is a hydroxyl group and $R^2$, $R^3$, $R^4$ and $R^5$ are selected from the group consisting of hydrogen atoms and hydroxyl groups.

**3.** A compound according to claim 1, wherein $R^2$ is a hydroxyl group and $R^1$, $R^3$, $R^4$ and $R^5$ are selected from the group consisting of hydrogen atoms and hydroxyl groups.

**4.** A compound according to claim 1, wherein $R^5$ is a hydroxyl group and $R^1$, $R^2$, $R^3$ and $R^4$ are selected from the group consisting of hydrogen atoms and hydroxyl groups.

**5.** A compound according to claim 1, wherein $R^3$ is a hydroxyl group and $R^1$, $R^2$, $R^4$ and $R^5$ are selected from the group consisting of hydrogen atoms and hydroxyl groups.

**6.** A compound according to claim 1, wherein $R^4$ is a hydroxyl group and $R^1$, $R^2$, $R^3$ and $R^5$ are selected from the group consisting of hydrogen atoms and hydroxyl groups.

**7.** A compound according to any one of the preceding claims, wherein only one of $R^1$, $R^2$, $R^3$, $R^4$ and $R^5$ is a hydroxyl group and the rest are hydrogen atoms.

**8.** A compound according to anyone of claims 1 to 7 the compound has the formula:

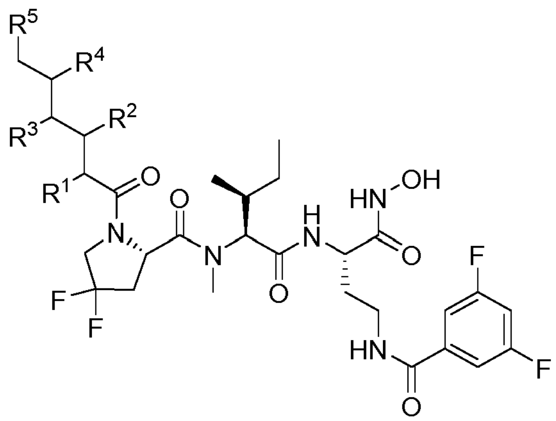

(II)

wherein each one of $R^1$, $R^2$, $R^3$, $R^4$ and $R^5$ is as defined in anyone of claims 1 to 7, or a pharmaceutically acceptable salt thereof or stereoisomer thereof.

**9.** A compound according to claim 1 selected from the group consisting of:

- N-(1-((4-(3,5-difluorobenzamido)-1-(hydroxyamino)-1-oxobutan-2-yl)amino)-3-methyl-1-oxopentan-2-yl)-4,4-difluoro-1 -(2-hydroxyhexanoyl)-N-methylpyrrolidine-2-carboxamide
- N-(1-((4-(3,5-difluorobenzamido)-1-(hydroxyamino)-1-oxobutan-2-yl)amino)-3-methyl-1-oxopentan-2-yl)-4,4-difluoro-1 -(3-hydroxyhexanoyl)-N-methylpyrrolidine-2-carboxamide
- N-(1-((4-(3,5-difluorobenzamido)-1-(hydroxyamino)-1-oxobutan-2-yl)amino)-3-methyl-1-oxopentan-2-yl)-4,4-difluoro-1 -(4-hydroxyhexanoyl)-N-methylpyrrolidine-2-carboxamide
- N-(1-((4-(3,5-difluorobenzamido)-1-(hydroxyamino)-1-oxobutan-2-yl)amino)-3-methyl-1-oxopentan-2-yl)-4,4-difluoro-1 -(5-hydroxyhexanoyl)-N-methylpyrrolidine-2-carboxamide
- N-(1-((4-(3,5-difluorobenzamido)-1-(hydroxyamino)-1-oxobutan-2-yl)amino)-3-methyl-1-oxopentan-2-yl)-4,4-difluoro-1 -(6-hydroxyhexanoyl)-N-methylpyrrolidine-2-carboxamide

or a pharmaceutically acceptable salt or stereoisomer thereof.

**10.** A compound according to claim 9 selected from the group consisting of:

- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1 -(2-hydroxyhexanoyl)-N-methylpyrrolidine-2-carboxamide
- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1 -(3-hydroxyhexanoyl)-N-methylpyrrolidine-2-carboxamide
- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1 -(4-hydroxyhexanoyl)-N-methylpyrrolidine-2-carboxamide
- (2S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1 -(5-hydroxyhexanoyl)-N-methylpyrrolidine-2-carboxamide
- (S)-N-((2S,3S)-1-(((S)-4-(3,5-difluorobenzamido)-1-(hydroxyamino)-1-oxobutan-2-yl)amino)-3-methyl-1-oxopentan-2-yl)-4,4-difluoro-1 -(6-hydroxyhexanoyl)-N-methylpyrrolidine-2-carboxamide

or a pharmaceutically acceptable salt thereof.

**11.** Pharmaceutical compositions comprising a compound according to anyone of claims 1 to 10 and a pharmaceutically acceptable carrier, adjuvant or vehicle.

**12.** A compound according to anyone of claims 1 to 10 or a pharmaceutical composition according to claim 11, for use as a medicament.

**13.** A compound according to anyone of claims 1 to 10 for use in the prevention and/or treatment of epilepsy, schizophrenia, Alzheimer disease, autism (in particular associated to fragile X syndrome), mental retardation, bipolar disorders, mood disorders such as bipolar disorders, depression, vascular diseases such as ischemic stroke and atherosclerosis, inflammatory diseases such as multiple sclerosis and rheumatoid arthritis, drug addiction, neuropathic pain, lung diseases such as asthma and chronic obstructive pulmonary disease, cancer and sepsis.

Europäisches Patentamt
European Patent Office
Office européen des brevets

# EUROPEAN SEARCH REPORT

Application Number
EP 24 38 3019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2017/085034 A1 (IPROTEOS S L [ES]) 26 May 2017 (2017-05-26)<br>* The whole document; claims; examples 7, 9, 10 *<br>----- | 1,4,7-13<br>2,3,5,6 | INV.<br>C07D207/16<br>A61K31/401<br>A61P9/10<br>A61P11/00<br>A61P11/06<br>A61P19/02<br>A61P21/00<br>A61P25/04<br>A61P25/08<br>A61P25/18<br>A61P25/24<br>A61P25/28<br>A61P25/30<br>A61P31/00<br>A61P35/00 |
| Y | AL-AWADHI FATMA H. ET AL: "Discovery, Synthesis, Pharmacological Profiling, and Biological Characterization of Brintonamides A-E, Novel Dual Protease and GPCR Modulators from a Marine Cyanobacterium", JOURNAL OF MEDICINAL CHEMISTRY, vol. 61, no. 14, 17 July 2018 (2018-07-17), pages 6364-6378, XP093246093, US ISSN: 0022-2623, DOI: 10.1021/acs.jmedchem.8b00885 Retrieved from the Internet: URL:https://pubs.acs.org/doi/pdf/10.1021/acs.jmedchem.8b00885><br>* page 6364 - page 6366; figure 2; compounds 1, 2 *<br>----- | 2,3,5,6 | TECHNICAL FIELDS SEARCHED (IPC)<br>A61P<br>A61K<br>C07D |

The present search report has been drawn up for all claims

1

| Place of search | Date of completion of the search | Examiner |
|---|---|---|
| Munich | 7 February 2025 | Sotoca Usina, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 38 3019

This annex lists the patent family members relating to the patent documents cited in the above-mentioned European search report. The members are as contained in the European Patent Office EDP file on
The European Patent Office is in no way liable for these particulars which are merely given for the purpose of information.

07-02-2025

| Patent document cited in search report | Publication date | Patent family member(s) | Publication date |
|---|---|---|---|
| WO 2017085034 A1 | 26-05-2017 | AU 2016356876 A1 | 10-05-2018 |
| | | BR 112018009641 A2 | 06-11-2018 |
| | | CA 3002892 A1 | 26-05-2017 |
| | | CN 108290925 A | 17-07-2018 |
| | | EA 201891184 A1 | 30-11-2018 |
| | | EP 3377515 A1 | 26-09-2018 |
| | | ES 2766548 T3 | 12-06-2020 |
| | | HK 1255029 A1 | 02-08-2019 |
| | | IL 259286 A | 31-07-2018 |
| | | JP 6795606 B2 | 02-12-2020 |
| | | JP 2018535258 A | 29-11-2018 |
| | | KR 20180071388 A | 27-06-2018 |
| | | NZ 741898 A | 23-02-2024 |
| | | US 2018319839 A1 | 08-11-2018 |
| | | WO 2017085034 A1 | 26-05-2017 |
| | | ZA 201803212 B | 26-08-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017085034 A1

### Non-patent literature cited in the description


- **YAMAMORI H et al.** *Neurosci Lett.*, 2013, vol. 556, 37-41
- **KONOPKA A et al.** *Epilepsy Res.*, 2013, vol. 1-2, 45-58
- **LI S et al.** *Brain Res*, 2012, vol. 1453, 46-55
- **BROEKAART DW et al.** *Neuropathol Appl Neurobiol.*, 2020, 142-159
- **PIJET B et al.** *Mol Neurobiol.*, 2018, vol. 55, 9294-9306
- **LEIGH M J et al.** *J Dev Behav Pediatr*, 2013, vol. 34, 1849-1857
- **ABDALLAH M W et al.** *Autism Res*, 2012, vol. 5, 428-433
- **HAN H et al.** *Psychiatry Res*, 2011, vol. 190, 163-174
- **SAMOCHOWIEC A et al.** *Brain Res*, 2010, vol. 1327, 103-6
- **RYBAKOWSKI J K et al.** *J Affect Disord*, 2013 (146), 286-289
- **YOSHIDA T et al.** *PLoS One*, 2012, vol. 7, e42676
- **FRAGKOULI A et al.** *J Neurochem*, 2012, vol. 121, 239-251
- **MIZOGUCHI et al.** *J Pharmacol Exp*, 2009 (331), 14-22
- **CHATURVEDI M et al.** *Mol. Neurobiol.*, 2014, vol. 49, 563-573
- **KAWASAKI Y et al.** *Nature Medicine*, 2008, vol. 3, 331-336
- **OPDENAKKER G et al.** *Lancet Nurol.*, 2003, vol. 2, 747-756
- **GIJBELS K et al.** *J. Neurosci. Res.*, 1993, vol. 36, 432-440
- **HEWSON A et al.** *Inflamm. Res.*, 1995, vol. 44, 345-349
- **AGRAWAL S et al.** *J. Exp. Med.*, 2006, vol. 203, 1007-1019
- **JANSSENS S et al.** *Cardiovsac. Res.*, 2006, vol. 69, 585-594
- **TAYEBJEE M H et al.** *Curr. Med. Chem.*, 2005, vol. 12, 917-925
- **LI M et al.** *J. Int. Med. Res.*, 2014, vol. 1, 224-230
- **NEWMAN K M et al.** *Arteriosclerosis and thrombosis: a journal of vascular biology*, 1994, vol. 8, 1315-1320
- **LINDEMAN J H et al.** *Circulation*, 2009, vol. 119, 2209-2216
- **MATSUMURA S et al.** *J. Clin. Invest.*, 2005, vol. 115, 559-609
- **KESSENBROCK K et al.** *Cell*, 2010, vol. 141, 52-67
- **COUSSENS L M et al.** *Science*, 2002, vol. 295, 2387-2392
- **BJORKLUND M et al.** *Biochim Biophys Acta*, 2005, vol. 1755, 37-69
- **DEMEDTS I K et al.** *Curr Opin Pharmacol*, 2005, vol. 5 (3), 257-63
- **KELLY EA et al.** *Am. J. Respir. Crit. Care Med.*, 2000, vol. 162, 1157-1161
- **XIE SS. et al.** *J Int Med Res.*, December 2014, vol. 42 (6), 1272-84